Europäisches Patentamt

European Patent Office

Office européen des brevets

(19)

(11) Veröffentlichungsnummer: **0 229 241**

**A2**

(12) ## EUROPÄISCHE PATENTANMELDUNG

(21) Anmeldenummer: 86114757.7

(22) Anmeldetag: 23.10.86

(51) Int. Cl.³: **A 61 B 8/00**

(30) Priorität: 18.12.85 DE 3544811

(43) Veröffentlichungstag der Anmeldung:
22.07.87 Patentblatt 87/30

(84) Benannte Vertragsstaaten:
FR GB IT

(71) Anmelder: DORNIER MEDIZINTECHNIK GMBH
Postfach 1128
D-8034 Germering 1(DE)

(72) Erfinder: Grözinger, Reiner, Dipl.-Ing.
Greppenstrasse 9
D-8031 Alling(DE)

(72) Erfinder: Isdebski, Kai
Bogenweilerstrasse 27
D-7968 Saulgau(DE)

(72) Erfinder: Wess, Othmar, Dr. Dipl.-Phys.
Max-Nadler-Strasse 17
D-8000 München 81(DE)

(72) Erfinder: Windsheimer, Manfred, Dipl.-Ing.
Ludwigstrasse 2
D-8034 Germering(DE)

(72) Erfinder: Erhardt, Wolfgang, Dipl.-Ing.
Landsberger Strasse 61
D-8080 Fürstenfeldbruck(DE)

(74) Vertreter: Landsmann, Ralf, Dipl.-Ing.
Kleeweg 3
D-7990 Friedrichshafen 1(DE)

(54) Vorrichtung zur Ankopplung einer Membran an die Haut des menschlichen Körpers.

(57) Gegenstand der Erfindung ist eine Vorrichtung zur Ankopplung einer Membran an die Haut des menschlichen Körpers, wobei sich zwischen Membran und Haut eine Gelschicht (24) befindet und auf der der Gelschicht abgewandten Seite der Membran (4) eine Wischereinrichtung (14, 16, 18; 32) vorhanden ist.

Fig. 1

EP 0 229 241 A2

DORNIER MEDIZINTECHNIK GMBH
Postfach 1128
8034 Germering 1

Reg. M 106 EU

Die Erfindung betrifft eine Vorrichtung zur Ankopplung einer Membran an die Haut des menschlichen Körpers.

Bei der medizinischen Diagnose und Therapie mittels medizintechnischer Geräte ist ist es häufig notwendig, Strahlung oder mechanische Wellen verlustfrei in den Körper des Patienten einzukoppeln.

Dazu werden eventuelle Körperhaare durch Rasur entfernt und eine Gelschicht auf die Haut aufgetragen. Wird nun die Membran des Behandlungsgeräts gegen die Gelschicht gedrückt, so kann sich eine luftspaltlose Ankopplung ergeben, jedoch besteht die Gefahr, dass Luftblasen eingeschlossen werden, die den Behandlungserfolg vermindern oder unmöglich machen.

Der Arzt oder Medizintechniker können eine persönliche handwerkliche Geschicklichkeit bei der Ankopplung von Patienten erwerben, die sich jedoch in der Regel nicht auf andere Personen übertragen lässt.

Es wurde deshalb z.B. schon vorgeschlagen, den Raum zwischen der Haut des Patienten und der Membran zu evakuieren, wobei jedoch die Gefahr besteht, dass isolierte Luftblasen erhalten bleiben.

Aus den vorgenannten Gründen ist bisher zum Beispiel eine erfolgreiche berührungsfreie Lithotripsie mittels Stosswellen dann möglich, wenn der Patient in einer Wanne mit entgastem Wasser behandelt wird, weil dadurch alle Probleme der Ankoppelung gelöst sind.

Der Erfindung liegt die Aufgabe zugrunde, eine Vorrichtung zu schaffen, mit deren Hilfe auch ungeübtes Klinikpersonal einen Patienten luftspaltlos an die Membran eines Behandlungsgeräts ankoppeln kann.

Diese Aufgabe wird erfindungsgemäss dadurch gelöst, dass sich auf der Innenseite der Membran eine verschiebbare Wischeinrichtung befindet, mit der die Membran gegen den gelbeschichteten Körper eines Patienten angewischt werden kann.

Bei dieser Art der Technik werden zugleich Druck und Zug auf ein mit Klebstoff versehenes flächenhaftes Element angewendet, um eine gute Verbindung mit seiner Unterlage herzustellen. Nach diesem Prinzip werden zum Beispiel Tapeten oder Plakate angeklebt, wobei in der Regel mittels Bürsten von innen nach aussen gestrichen wird und eventuelle Lufteinschlüsse zum Rand befördert werden. Es entsteht ein gleichmässiger Klebstoffilm, der das Papier auf der gesamten Fläche mit der Unterlage verbindet.

Weitere Ausgestaltungen der Erfindung sind Gegenstand von Unteransprüchen.

Die Erfindung wird nachfolgend anhand von Figuren näher erläutert.

Es zeigen:
Fig. 1 eine in einem Rahmen befindliche Membran mit erfindungsgemässer Wischeinrichtung,
Fig. 2 und 3 verschiedene Ausführungsformen für einen Wischer,
Fig 4 eine Schnittansicht von Fig. 1 und
Fig. 5 eine weitere Ausgestaltung der Erfindung.

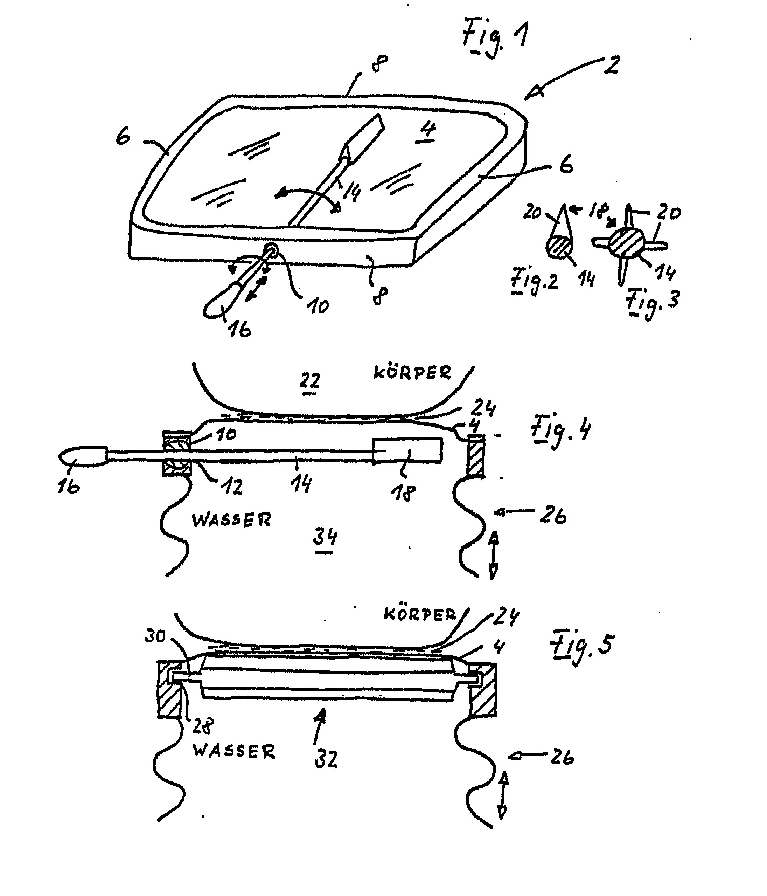

Fig. 1 zeigt einen Rahmen 2 eines Behandlungsgeräts, wobei in die Stirnwände 6 und Seitenwände 8 des Rahmens eine elastische Membran 4 eingespannt ist. An einer Stirnwand 6 oder Seitenwand 8 des Rahmens befindet sich ein Gelenklager 10, in dessen Bohrung 12 (Fig. 4) eine Stange 14 schwenkbar und in ihrer Längsrichtung verschiebbar und um ihre Längsachse drehbar gelagert ist. Die Stange 14 weist an einem Ende einen Handgriff 16 und am anderen Ende ein Wischer 18 auf. Der Wischer 18 kann eine oder mehrere Wischerlippen 20 aufweisen, wie in den Fig. 2 und 3 gezeigt. Der Wischer 18 kann auch eine Bürste sein, dies ist in den Fig. nicht gezeigt.

Fig 4 zeigt eine Schnittansicht von Fig. 1, wobei eine zwischen der Haut eines Körpers 22 und der Membran 4 befindliche Gelschicht 24 zu sehen ist. Am Rahmen 2 befindet sich ein Faltenbalg 26, zur höhenverstellbaren Anpassung der Membran 4 an den Körper 22.

Der Faltenbalg 26 ist auch in Fig. 5 dargestellt. Hier befinden sich in Ausnehmungen 28 des Rahmens 2 die Zapfen 30 einer Wischerwalze 32. Die Wischerwalze kann motorisch oder von Hand entlang des Rahmens 2 bewegt werden, wobei die aus elastischem Material bestehenden Wischerlippen unter Drehung für ein luftspaltloses Anlegen der Membran 4 an den Körper des Patienten sorgen. Eine oder mehrere Wischerwalzen können auch von der Rahmenmitte her zu den Wänden bewegt werden. Es ist auch möglich, dass die Wischerlippen oder die Wischerwalze aufblasbar gestaltet werden oder dass die Walze vibriert, um Luftblasen aus der Gelschicht 24 zu entfernen.

Funktion.

Der Körper 22 eines Patienten wird mittels einer nicht gezeigten Patientenliege auf die gelbeschichtete Membran 4 abgesenkt, die mittels des Faltenbalgs 26 höhenverstellbar ist. Die Membran bildet den Abschluss eines wassergefüllten Raumes 34, an dessen anderem Ende sich eine nicht gezeigte Stosswellenquelle oder ein Gerät zur Strahlenbehandlung befindet. Im Raum 34 herrscht geringer Überdruck. Mittels der Wischereinrichtung 16, 14, 18 kann nun von Hand die Membran durch Wischbewegungen an den Körper des Patienten angelegt werden, wobei sämtliche Lufblasen aus der Gelschicht 24 entfernt werden. Die Freiheitsgrade der Wischerbewegung sind durch Pfeile in Fig. 1 angedeutet. Beim Gegenstand von Fig. 5 sorgt die Bewegung der Wischerwalze für ein Anlegen der Membran an den Körper und für das Heraustreiben von Lufteinschlüssen.

21.10.86
PaL

DORNIER MEDIZINTECHNIK GMBH
Postfach 1128
8034 Germering 1

0229241

Reg. M 106 EU

Patentansprüche

1. Vorrichtung zur Ankopplung einer Membran an die Haut des menschlichen Körpers, wobei sich zwischen Membran und Haut eine Gelschicht befindet, dadurch gekennzeichnet, dass sich auf der der Gelschicht (24) abgewandten Seite der Membran (4) eine Wischereinrichtung (14, 16, 18; 32) befindet.

2. Vorrichtung nach Anspruch 1, dadurch gekennzeichnet, dass die Stange (14) der Wischereinrichtung in einem Gelenklager (10) in der Wand (6, 8) eines Rahmens (2) gelagert ist.

3. Wischereinrichtung nach Anspruch 1, dadurch gekennzeichnet, dass die Stange (14) im Gelenklager (10) drehbeweglich und in Längsrichtung verschiebbar gelagert ist.

4. Wischereinrichtung nach Ansprüchen 1 - 3, dadurch gekennzeichnet, dass das Wischerblatt (18) ein oder mehrere Wischerlippen (20) aufweist.

5. Wischereinrichtung nach Ansprüchen 1- 4, dadurch gekennzeichnet, dass das Wischerblatt (18) rotatorisch antreibbar ist.

6. Wischereinrichtung nach Anspruch 1, dadurch gekennzeichnet, dass mindestens eine im Rahmen (2) translatorisch bewegbare, um ihre Achse rotierbare Wischerwalze (34) vorhanden ist.

21.10.86
PaL

*Fig. 1*

8

6

4

2

14

10

16

*Fig.2*  20  18  20  *Fig.3*

14  14

KÖRPER

22

24

4

*Fig.4*

10

16  12  14  18

WASSER

34

26

KÖRPER  24

30  4  *Fig.5*

28

WASSER  32  26